# EUROPEAN PATENT SPECIFICATION

(11) **EP 3 533 385 B1**
(45) Date of publication and mention of the grant of the patent: **27.03.2024**
(21) Application number: 17866317.5
(22) Date of filing: 25.10.2017
(51) Int. Cl.: A61B 5/00, A63B 69/00, A61B 5/145

(54) **EXERCISE ASSISTANCE DEVICE, EXERCISE ASSISTANCE SYSTEM, EXERCISE ASSISTANCE METHOD, AND NON-TRANSITIVE SUBSTANTIVE RECORDING MEDIUM**
TRAININGSASSISTENZVORRICHTUNG, TRAININGSASSISTENZSYSTEM, TRAININGSASSISTENZVERFAHREN UND NICHTTRANSITIVES SUBSTANZIELLES AUFZEICHNUNGSMEDIUM
DISPOSITIF D'AIDE À L'EXERCICE, SYSTÈME D'AIDE À L'EXERCICE, PROCÉDÉ D'AIDE À L'EXERCICE ET SUPPORT D'ENREGISTREMENT MATÉRIEL NON TRANSITOIRE

(30) Priority: 26.10.2016 JP 2016209773
(43) Date of publication of application: 04.09.2019
(73) Proprietor: JSR Corporation, Tokyo 105-8640 (JP)
(72) Inventor: FUJIOKA, Masayasu, Tokyo 105-8640 (JP); MIYASAKO, Takaaki, Tokyo 105-8640 (JP); HAMADA, Kenichi, Tokyo 105-8640 (JP); HAYASHI, Taito, Tokyo 105-8640 (JP)
(74) Representative: TBK
(86) International application number: PCT/JP2017/038508
(87) International publication number: WO 2018/079601

(56) References cited:
- WO-A1-2016/017616
- WO-A1-2016/025430
- JP-A- 2004 038 990
- JP-A- 2014 130 096
- JP-A- 2015 058 364
- JP-A- 2015 157 029
- JP-A- 2016 126 622
- US-A1- 2007 083 092
- US-A1- 2010 234 184
- US-A1- 2014 323 820
- US-A1- 2015 330 893
- US-A1- 2016 066 835
- US-A1- 2016 275 805

## Description

### CROSS-REFERENCE TO RELATED APPLICATIONS

This international application claims the benefit of Japanese Patent Application No. 2016-209773 filed on October 26, 2016 with the Japan Patent Office.

### TECHNICAL FIELD

The present disclosure relates to an exercise assistance device, an exercise assistance system, an exercise assistance method, and a non-transitory tangible storage medium.

### BACKGROUND ART

Patent Document 1 discloses the following technique: to detect a user's biological information such as pulse rate, blood pressure, and body temperature, before exercise; to compare the detected biological information with biological information under normal conditions, to thereby determine the user's physical condition before exercise; and to correct an exercise menu according to a determination result of the physical condition.

Further prior art can be found in document US 2016 / 0 066 835 A1 describing a non-invasive biofeedback system, in document US 2007 / 0 083 092 A1 describing an external exercise monitor, in document US 2010 / 0 234 184 A1 describing a method and an apparatus for controlling physical exertion as well as in document WO 2016 / 017 616 A1 describing an exercise support device and an exercise support method.

### PRIOR ART DOCUMENTS

### PATENT DOCUMENTS

Patent Document 1: JP 2015 - 157 029 A

### SUMMARY OF THE INVENTION

### PROBLEMS TO BE SOLVED BY THE INVENTION

The biological information such as pulse rate, blood pressure, and body temperature, varies depending on various factors. Thus, it may be impossible, in some cases, to set an appropriate exercise menu based on the biological information.

In one aspect of the present disclosure, it is desirable to provide an exercise assistance device, an exercise assistance system, an exercise assistance method, and a non-transitory tangible storage medium that enable appropriate setting of an exercise menu based on a user's condition.

### MEANS FOR SOLVING THE PROBLEMS

One aspect of the present disclosure is an exercise assistance device according to claim 1. Further advantageous modifications thereof are defined in the dependent claims. The exercise assistance device according to one aspect of the present disclosure enables appropriate setting of the exercise menu according to a user's condition.

Another aspect of the present disclosure is an exercise assistance system according to independent clairr 9. The exercise assistance system according to another aspect of the present disclosure enables appropriate setting of the exercise menu according to a user's condition.

Another aspect of the present disclosure is an exercise assistance method according to independent clairr 10. The exercise assistance method according to another aspect of the present disclosure enables appropriate setting of the exercise menu according to a user's condition.

Another aspect of the present disclosure is a non-transitory tangible storage medium according to independent clairr 11. The non-transitory tangible storage medium according to another aspect of the present disclosure enables appropriate setting of the exercise menu according to a user's condition.

### BRIEF DESCRIPTION OF THE DRAWINGS

FIG. 1 is a block diagram showing an example configuration of an exercise assistance system.
FIG. 2 is a block diagram showing an example of a functional configuration of a controller.
FIG. 3 is a block diagram showing an example configuration of a biosensor.
FIG. 4A is a block diagram showing an example configuration of a body temperature sensor, FIG. 4B is a block diagram showing an example configuration of an air temperature sensor, and FIG. 4C is a block diagram showing an example configuration of a secretion amount sensor.
FIG. 5A is a block diagram showing an example configuration of a heart rate sensor, and FIG. 5B is a block diagram showing an example configuration of an acceleration sensor.
FIG. 6 is a flowchart showing the entirety of an example of a process performed by an exercise assistance device.
FIG. 7 is an explanatory diagram showing an example of an exercise menu database.
FIG. 8 is a flowchart showing an example of a correction value determination process performed by the exercise assistance device.
FIG. 9A is an explanatory diagram showing an example of a relationship between body temperature and a correction value ΔY1 specified by a map, FIG. 9B is an explanatory diagram showing an example of a relationship between air temperature and a correction value ΔY2 specified by a map, and FIG. 9C is an explanatory diagram specifying an example of a relationship between a secretion amount and a correction value ΔY3 specified by a map.
FIG. 10A is an explanatory diagram showing an example of a relationship between heart rate and a correction value ΔY4 specified by a map, and FIG. 10B is an explanatory diagram showing an example of a relationship between an acceleration and a correction value ΔY5 specified by a map.
FIG. 11 is a flowchart showing an example of a process performed by the biosensor.
FIG. 12 is an explanatory diagram showing another mode of the exercise menu database.
FIG. 13 is an explanatory diagram showing another mode of the exercise menu database.
FIG. 14 is an explanatory diagram showing another mode of the exercise menu database.
FIG. 15 is an explanatory diagram showing another mode of the exercise menu database.

### EXPLANATION OF REFERENCE NUMERALS

1...exercise assistance system, 3...exercise assistance device, 5...biosensor, 7...body temperature sensor, 9...air temperature sensor, 11...secretion amount sensor, 13...heart rate sensor, 15...acceleration sensor, 17...controller, 19...inputter, 21...communicator, 23...exercise menu database storage, 25...time measurer, 27...display, 29...CPU, 31...memory, 33...biological information acquisition unit, 35... exercise menu setting unit, 37...exercise menu presentation unit, 39...correction information acquisition unit, 41...database creation unit, 43...sensor body, 45...controller, 47...communicator, 49...base material layer, 51 ... working electrode, 53...reference electrode, 55...counter electrode, 57...electron transfer mediator layer, 59...enzyme layer, 61...absorber layer, 63...CPU, 65...memory, 67...body temperature measurer, 69, 75, 81, 87, 93...controller, 71, 77, 83, 89, 95... communicator, 73...air temperature measurer, 79...secretion amount measurer, 85...heart rate measurer, 91 ...acceleration measurer

### MODE FOR CARRYING OUT THE INVENTION

An example embodiment of the present disclosure will be described with reference to the drawings.

### <First Embodiment>

### 1. Overall Configuration of Exercise Assistance System 1

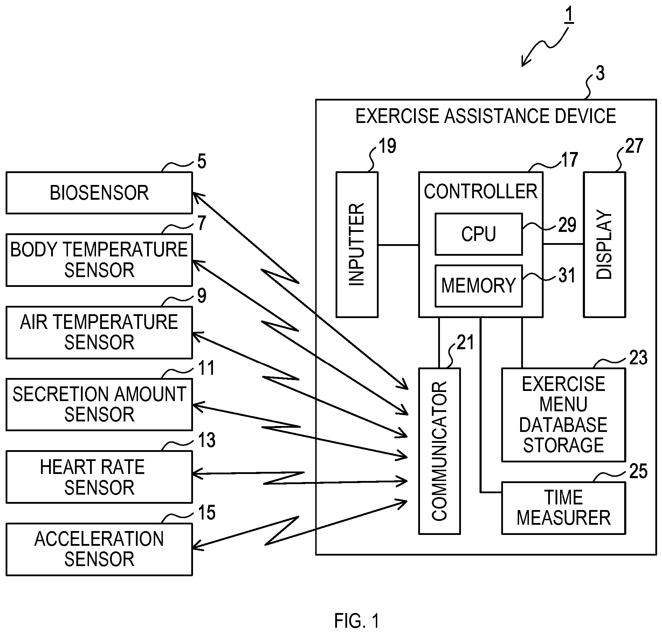

A configuration of an exercise assistance system 1 will be described with reference to FIG. 1 to FIG. 5. As shown in FIG. 1, the exercise assistance system 1 comprises an exercise assistance device 3, a biosensor 5, a body temperature sensor 7, an air temperature sensor 9, a secretion amount sensor 11, a heart rate sensor 13, and an acceleration sensor 15. The body temperature sensor 7, the air temperature sensor 9, the secretion amount sensor 11, the heart rate sensor 13, and the acceleration sensor 15 correspond to a sensor for correction. Hereinafter, the body temperature sensor 7, the air temperature sensor 9, the secretion amount sensor 11, the heart rate sensor 13, and the acceleration sensor 15 are also referred to collectively as the sensor for correction.

### 2. Configuration of Exercise Assistance Device 3

The exercise assistance device 3 may be, for example, a portable information terminal device. The exercise assistance device 3 comprises a controller 17, an inputter 19, a communicator 21, an exercise menu database storage 23, a time measurer 25, and a display 27.

The controller 17 is mainly configured with a known microcomputer comprising a CPU 29 and a semiconductor memory (hereinafter referred to as a memory 31) such as a RAM, a ROM, and a flash memory. Various functions of the controller 17 are performed by the CPU 29 executing a program or programs stored in a non-transitory tangible storage medium. In this example, the memory 31 corresponds to the non-transitory tangible storage medium in which the program or programs are stored.

Biological information transmitted by the biosensor 5 is received by the communicator 21. The biological information is information indicating a result of measurement by the biosensor 5. The biological information includes information indicating a result of measurement of a measurement target substance. The result of measurement of the measurement target substance includes a lactic acid concentration.

The memory 31 stores the information received by the communicator 21. The communicator 21 receives information (hereinafter referred to as correction information) transmitted by the body temperature sensor 7, the air temperature sensor 9, the secretion amount sensor 11, the heart rate sensor 13, the acceleration sensor 15, and so on. The memory 31 stores the correction information in association with the biological information. The correction information will be described later. The memory 31 corresponds to a storage unit. The microcomputer constituting the controller 17 may be one or more in number.

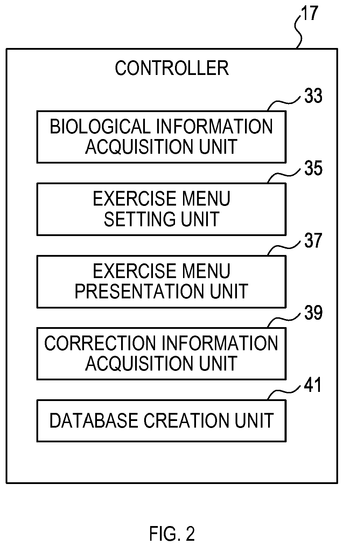

As shown in FIG. 2, the controller 17 comprises, as functional elements realized by execution of the program or programs by the CPU 29, a biological information acquisition unit 33, an exercise menu setting unit 35, an exercise menu presentation unit 37, a correction information acquisition unit 39, and a database creation unit 41.

The biological information acquisition unit 33 acquires the biological information from the biosensor 5, and stores the biological information in the memory 31. The exercise menu setting unit 35 sets an exercise menu based on the biological information. The exercise menu presentation unit 37 presents the exercise menu set by the exercise menu setting unit 35.

The controller 17 may comprise hardware, such as an electronic circuit, instead of the microcomputer or in addition to the microcomputer. The electronic circuit may comprise at least one of a digital circuit or an analog circuit.

The inputter 19 receives an input operation by a user. The inputter 19 comprises, for example, a keyboard, a touch panel, a push button, and/or the like. Examples of contents to be inputted through the inputter 19 may include a below-described input of start of the exercise and a below-described request for presentation of the exercise menu. Further examples of the contents to be inputted through the inputter 19 may include the user's physical information, lifestyle information, management ID, and password. Examples of the physical information may include height, weight, body fat percentage, age, sex, and medical history. Examples of the lifestyle information may include presence/absence of smoking habit, frequency and amount of smoking, sleeping time, presence/absence of drinking habit, and frequency and amount of drinking. The inputter 19 corresponds to an input unit. The information to be inputted through the inputter 19 corresponds to input information.

The communicator 21 performs communication with the biosensor 5, the body temperature sensor 7, the air temperature sensor 9, the secretion amount sensor 11, the heart rate sensor 13, and the acceleration sensor 15 (hereinafter also referred to collectively as a sensor group). A preferred communication method is wireless communication.

The exercise menu database storage 23 is a memory that stores an exercise menu database. The exercise menu database will be described below. The time measurer 25 outputs time information, which is information on the clock time at a time point when the biological information is measured.

The display 27 is a member that can display an image. Examples of the display 27 may include a liquid crystal display and an organic EL display.

### 3. Configuration of Sensor Group

### (1) Configuration of Biosensor 5

The biosensor 5 is a sensor to measure a concentration of the measurement target substance contained in the user's secretion. The biosensor 5 is worn on a part of the user's body. The part on which the biosensor 5 is worn may be set as appropriate, and can be, for example, a wrist, a leg, a head, a chest, and an abdomen. Examples of the secretion from a living body may include perspiration, tears, saliva, and urine. The measurement target substance contained in the secretion from the living body includes actic acid and optionally one or more of glucose (grape sugar), ethanol, uric acid, urea, vitamins, amino acid, free cholesterol, and phosphate ion.

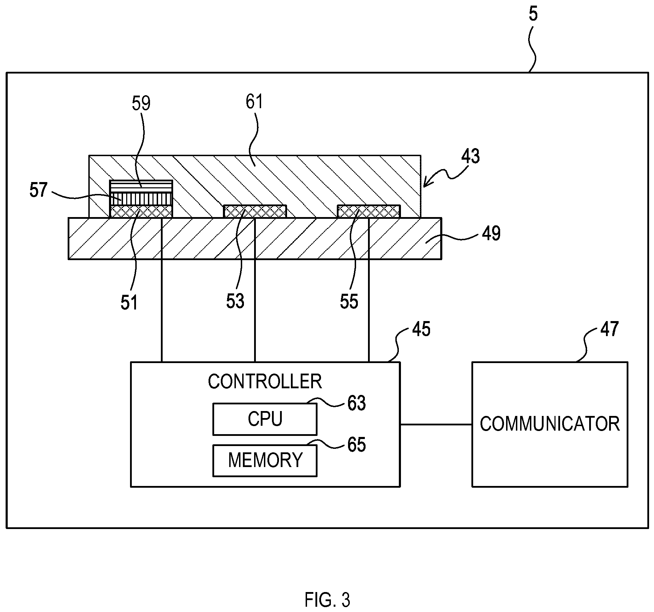

A configuration of the biosensor 5 will be described with reference to FIG. 3. The biosensor 5 comprises a sensor body 43, a controller 45, and a communicator 47. The sensor body 43 comprises a base material layer 49, a working electrode 51, a reference electrode 53, a counter electrode 55, an electron transfer mediator layer 57, an enzyme layer 59, and an absorber layer 61.

The base material layer 49 supports the working electrode 51, the reference electrode 53, and the counter electrode 55. The base material layer 49 is not limited in particular, as long as it supports the working electrode 51, the reference electrode 53, and the counter electrode 55. Examples of a material that can be used for the base material layer 49 may include: thermoplastic elastomers such as thermoplastic styrene-based elastomer, thermoplastic olefin-based elastomer, thermoplastic diene-based elastomer, thermoplastic vinyl chloride-based elastomer, thermoplastic urethane-based elastomer, and thermoplastic silicone-based elastomer; and thermoplastic resins such as polyethylene, polypropylene, polystyrene, polyethylene terephthalate, cellulose, acrylonitrile-styrene (AS) resin, glass epoxy, acrylonitrile-butadiene-styrene (ABS) resin, methacrylic resin, and vinyl chloride.

Among these, the thermoplastic elastomers are preferably used as the material for the base material layer 49 due to their excellent flexibility. The thickness of the base material layer 49 is preferably 5 µm or greater, more preferably 20 µm or greater, and yet more preferably 50 µm or greater. The thickness of the base material layer 49 is preferably 2 mm or less, more preferably 1 mm or less, and yet more preferably 500 µm or less. The material layer 49 is more excellent in supporting property and also more excellent in flexibility when the thickness thereof is within the above-described ranges.

The working electrode 51, the electron transfer mediator layer 57, the enzyme layer 59, and the absorber layer 61 are layered and arranged in this order on the base material layer 49. Shapes of the working electrode 51, the reference electrode 53, and the counter electrode 55 are each, for example, a circular shape such as a true circle or an ellipse, or a polygonal shape such as a triangle or a quadrangle. Diameters of the working electrode 51, the reference electrode 53, and the counter electrode 55 are each, for example, 1 mm or more and 5 cm or less when viewed from a direction orthogonal to the base material layer 49. Examples of an electrode-forming material that forms the working electrode 51 and the counter electrode 55 may include a material containing a carbon material, such as carbon, carbon nanotube, fullerene, or graphene. Examples of an electrode-forming material that forms the reference electrode 53 may include a material containing silver and silver chloride.

The electron transfer mediator layer 57 contains an electron transfer mediator. Examples of the electron transfer mediator may include ferrocene, quinones, ferrocenecarboxylic acid, potassium ferricyanide, osmium complex, ruthenium complex, phenothiazine derivative, phenazine methosulfate derivative, p-aminophenol, meldola blue, and 2,6-dichlorophenolindophenol.

The enzyme layer 59 contains an enzyme. Examples of the enzyme may include lactate oxidase (lactic acid oxidizing enzyme), glucose oxidase (glucose oxidizing enzyme), alkaline phosphatase, alcohol oxidase, uricase, L-amino-acid oxidase, urease, cholesterol oxidase, phosphatase, and horseradish peroxidase.

The absorber layer 61 covers the working electrode 51, the electron transfer mediator layer 57, the enzyme layer 59, the reference electrode 53, and the counter electrode 55. Examples of an electrode-forming material that forms the absorber layer 61 may include a hydrogel. The absorber layer 61 is positioned to be in contact with the user's skin when the biosensor 5 is in use. The absorber layer 61 can absorb perspiration.

The sensor body 43 works as below. When the biosensor 5 is in use, the absorber layer 61 is in contact with the user's skin. The absorber layer 61 absorbs the user's secretion. Examples of the secretion may include perspiration. The secretion absorbed by the absorber layer 61 passes through the absorber layer 61 and reaches the enzyme layer 59. The enzyme layer 59 produces an electrode-detectable substance or a precursor of electrode-detectable substance from the measurement target substance contained in the secretion, in the presence of the enzyme. Specifically, the enzyme layer 59 oxidizes, for example, lactic acid contained in perspiration in the presence of lactate oxidase to generate pyruvic acid.

Next, an electrochemical reaction, including an oxidation-reduction reaction, of the electron transfer mediator generally occurs in the electron transfer mediator layer 57, and an electrode-detectable substance is generated from the precursor of electrode-detectable substance. Then, the electrode-detectable substance generated in the electron transfer mediator layer 57 causes an electrochemical reaction including an oxidation-reduction reaction on a surface of the working electrode 51. This causes an electric current in the biosensor 5. The biosensor 5 electrically detects the concentration of the electrode-detectable substance. Consequently, an electric current corresponding to the concentration of the measurement target substance, such as lactic acid, is caused in the sensor body 43. The sensor body 43 electrically detects the concentration of the measurement target substance based on a current value of the electric current.

The controller 45 is a microcomputer comprising a CPU 63 and a memory 65. The current value of the electric current caused in the sensor body 43 is inputted to the controller 45 as a signal indicating the lactic acid concentration. The controller 45 controls the communicator 47. The communicator 47 performs wireless communication with the exercise assistance device 3.

### (2) Configuration of Body Temperature Sensor 7

The body temperature sensor 7 is a sensor to measure the user's body temperature. The body temperature sensor 7 is worn on a part of the user's body. The part on which the body temperature sensor 7 is worn may be set as appropriate, and can be, for example, a wrist, a leg, a head, a chest, and an abdomen.

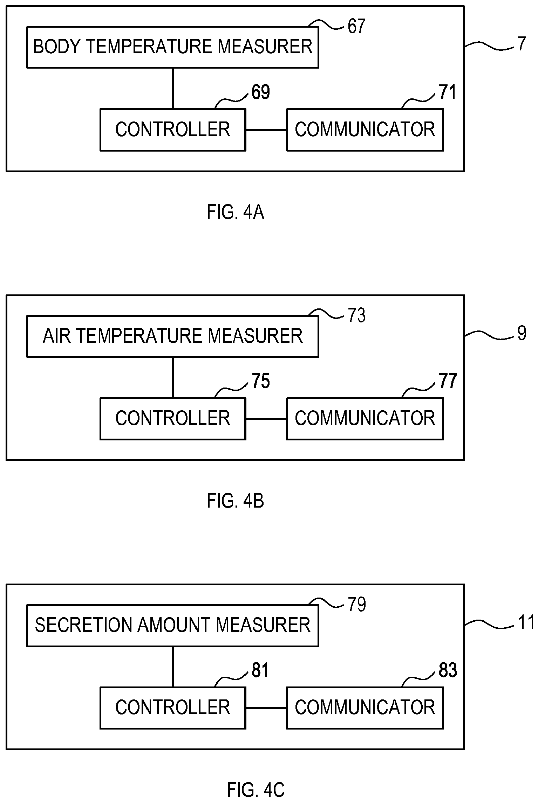

As shown in FIG. 4A, the body temperature sensor 7 comprises a body temperature measurer 67, a controller 69, and a communicator 71. The body temperature measurer 67 measures the user's body temperature, and outputs a signal indicating the body temperature to the controller 69. The controller 69 is a microcomputer. The controller 69 controls the communicator 71. The communicator 71 performs wireless communication with the exercise assistance device 3.

### (3) Configuration of Air Temperature Sensor 9

The air temperature sensor 9 is a sensor to measure an air temperature around the user. The air temperature sensor 9 is worn on a part of the user's body. The part on which the air temperature sensor 9 is worn may be set as appropriate, and can be, for example, a wrist, a leg, a head, a chest, and an abdomen. Alternatively, the air temperature sensor 9 may be attached to the exercise assistance device 3.

As shown in FIG. 4B, the air temperature sensor 9 comprises an air temperature measurer 73, a controller 75, and a communicator 77. The air temperature measurer 73 measures the air temperature, and outputs a signal indicating the air temperature to the controller 75. The controller 75 is a microcomputer. The controller 75 controls the communicator 77. The communicator 77 performs wireless communication with the exercise assistance device 3.

### (4) Configuration of Secretion Amount Sensor 11

The secretion amount sensor 11 is a sensor to measure an amount of a secretion secreted from the user. Examples of the secretion may include perspiration. The secretion amount sensor 11 is worn on a part of the user's body. The part on which the secretion amount sensor 11 is worn may be set as appropriate, and can be, for example, a wrist, a leg, a head, a chest, and an abdomen.

As shown in FIG. 4C, the secretion amount sensor 11 comprises a secretion amount measurer 79, a controller 81, and a communicator 83. The secretion amount measurer 79 measures a secretion amount of perspiration, and outputs a signal indicating the secretion amount to the controller 81. The secretion amount measurer 79, for example, also can measure an electrolyte concentration of sodium or the like in the perspiration to calculate the secretion amount of perspiration based on the numerical value of the measured electrolyte concentration, and also can measure electric conductivity and/or resistivity on a surface of skin to calculate the secretion amount of perspiration based on the numerical values of the measured electric conductivity and resistivity. The controller 81 is a microcomputer. The controller 81 controls the communicator 83. The communicator 83 performs wireless communication with the exercise assistance device 3.

### (5) Configuration of Heart Rate Sensor 13

The heart rate sensor 13 is a sensor to measure the user's heart rate. The heart rate sensor 13 is worn on a part of the user's body. The part on which the heart rate sensor 13 is worn may be set as appropriate, and can be, for example, a wrist, a leg, a head, a chest, and an abdomen.

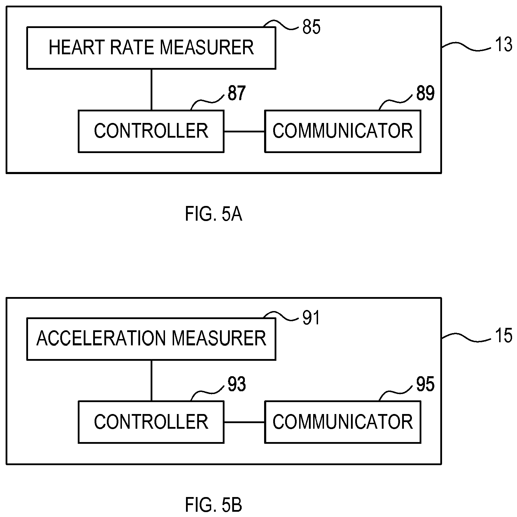

As shown in FIG. 5A, the heart rate sensor 13 comprises a heart rate measurer 85, a controller 87, and a communicator 89. The heart rate measurer 85 measures the heart rate, and outputs a signal indicating the heart rate to the controller 87. The controller 87 is a microcomputer. The controller 87 controls the communicator 89. The communicator 89 performs wireless communication with the exercise assistance device 3.

### (6) Configuration of Acceleration Sensor 15

The acceleration sensor 15 is a sensor to measure the user's acceleration. The acceleration sensor 15 is worn on a part of the user's body. The part on which the acceleration sensor 15 is worn may be set as appropriate, and can be, for example, a wrist, a leg, a head, a chest, and an abdomen.

As shown in FIG. 5B, the acceleration sensor 15 comprises an acceleration measurer 91, a controller 93, and a communicator 95. The acceleration measurer 91 measures the acceleration, and outputs a signal indicating the acceleration to the controller 93. The controller 93 is a microcomputer. The controller 93 controls the communicator 95. The communicator 95 performs wireless communication with the exercise assistance device 3.

### 4. Process Performed by Exercise Assistance Device 3

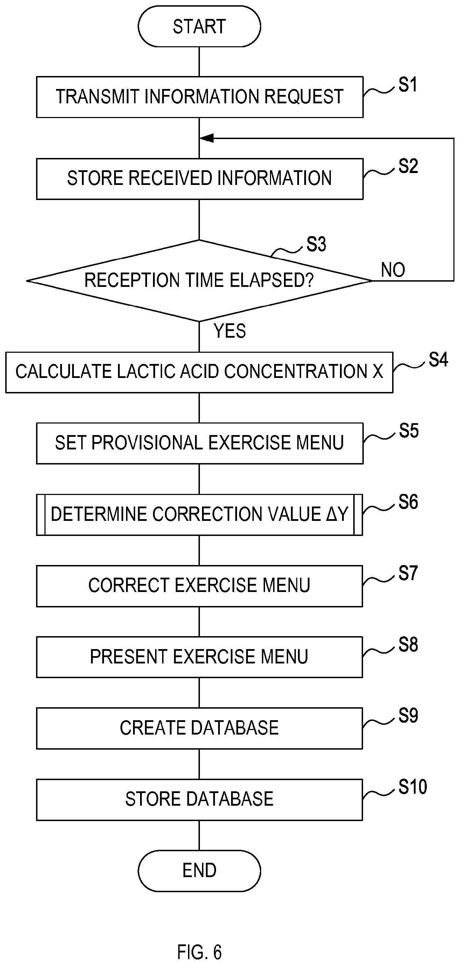

A process performed by the exercise assistance device 3 will be described with reference to FIG. 6 to FIG. 10. This process is performed when the user inputs, to the inputter 19, a request for presentation of the exercise menu.

In a step 1 in FIG. 6, the biological information acquisition unit 33 acquires the biological information from the biosensor 5, and the correction information acquisition unit 39 acquires correction information from the sensor for correction.

Specifically, the biological information acquisition unit 33 and the correction information acquisition unit 39 each transmit an information request to the corresponding sensor in the sensor group through the communicator 21. Upon receipt of this information request, the biosensor 5 transmits the biological information, and the sensor for correction transmits the correction information.

The correction information comprises body temperature information transmitted by the body temperature sensor 7, air temperature information transmitted by the air temperature sensor 9, secretion amount information transmitted by the secretion amount sensor 11, heart rate information transmitted by the heart rate sensor 13, and acceleration information transmitted by the acceleration sensor 15.

The body temperature information is information indicating a result of measurement of the body temperature. The air temperature information is information indicating a result of measurement of the air temperature. The secretion amount information is information indicating a result of measurement of the secretion amount. The heart rate information is information indicating a result of measurement of the heart rate. The acceleration information is information indicating a result of measurement of the acceleration.

The sensor group performs measurements repeatedly at specified intervals, and transmits information indicating the result of the respective measurements.

Further, in the present step 1, the biological information acquisition unit 33 instructs the time measurer 25 to output the time information. In response to the instruction, the time measurer 25 outputs the time information to the controller 17.

In a step 2, the biological information acquisition unit 33 receives the information transmitted by the sensor group through the communicator 21, and stores the information in the memory 31. Further, in the present step 2, the time information outputted by the time measurer 25 in the step 1 is stored in the memory 31. The memory 31 stores each piece of the received information and the time information in association with each other.

In a step 3, the biological information acquisition unit 33 determines whether a preset reception time has elapsed from a time point when the information request is transmitted in the step 1. If the reception time has elapsed, the process proceeds to a step 4. If the reception time has not elapsed yet, the process proceeds to the step 2.

In the step 4, the exercise menu setting unit 35 calculates the biological information, indicated by the biological information stored in the memory 31 in the step 2. According to an example not covered by the claimed invention, the biological information is the lactic acid concentration (hereinafter referred to as a lactic acid concentration X). If multiple pieces of the biological information are stored in the memory 31, the lactic acid concentration X is an average value of the lactic acid concentrations in the multiple pieces of the biological information. If the number of the pieces of the biological information stored in the memory 31 is only one, the lactic acid concentration X is the lactic acid concentration in the single piece of the biological information.

In a step 5, the exercise menu setting unit 35 provisionally sets the exercise menu based on the biological information, such as the lactic acid concentration X, calculated in the step 4.

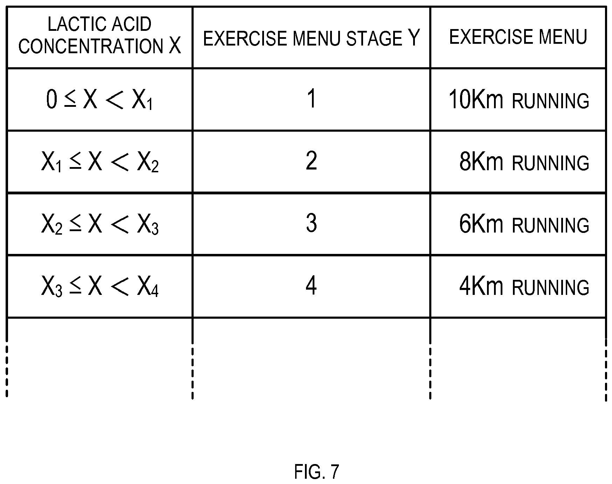

In a case where the exercise menu is provisionally set based on the lactic acid concentration X, the exercise menu is specifically set as below. The exercise menu setting unit 35 reads out the exercise menu database from the exercise menu database storage 23. As shown in FIG. 7, in the exercise menu database, the lactic acid concentration X, a time T, an exercise menu stage Y, and the exercise menu are associated with each other. The exercise menu setting unit 35 applies the lactic acid concentration X calculated in the step 4 to the exercise menu database, to thereby determine the exercise menu stage Y and the exercise menu. The time T is a period of time from a time point when the exercise is started to the present time point.

For example, when the lactic acid concentration X is 0 or more and less than X1, the exercise menu stage Y is 1. Further, for example, when the lactic acid concentration X is X1 or more and less than X2, the exercise menu stage Y is 2. In FIG. 7, a larger/smaller relationship of the numerical values is 0<Xl <X2<X3<X4.

The larger the value of the lactic acid concentration X is, the larger the value of the exercise menu stage Y is. The larger the value of the exercise menu stage Y is, the smaller the quantity and/or intensity of the exercise menu are. The quantity of the exercise menu is, for example, a distance the user moves during the exercise, an exercise time, or the like. A distance the user runs, a distance the user swims, and so on correspond to the quantity of the exercise menu. The intensity of the exercise menu is, for example, a load applied to the user during the exercise. For example, a load weight in muscle training, a running speed, a swimming speed, and so on correspond to the intensity of the exercise menu.

Described above is an example in which the lactic acid concentration X, the exercise menu stage Y, and the exercise menu are associated with each other in the exercise menu database. However, according to the invention a time TZ is used instead of the lactic acid concentration X. The time TZ is a period of time from the time point when the exercise is started to a time point when a second-order differential value of the lactic acid concentration with respect to the time first reaches a specified threshold. The time TZ corresponds to the biological information.

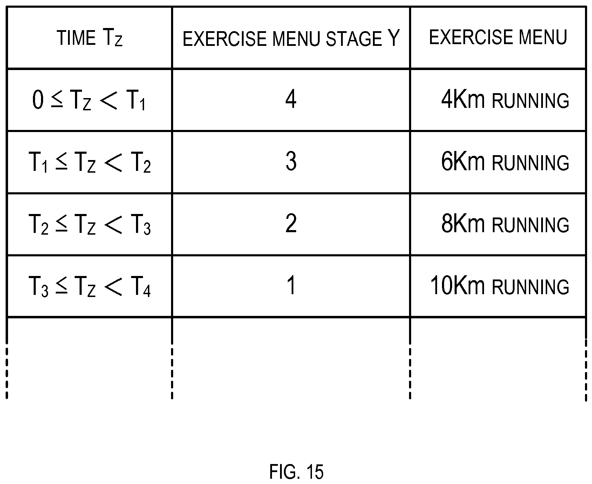

For example, as shown in FIG. 15, when the time TZ is 0 or more and less than T1, the exercise menu stage Y is 4. Further, for example, when the time TZ is T1 or more and less than T2, the exercise menu stage Y is 3. In FIG. 15 , a larger/smaller relationship of the numerical values is 0<T1<T2< T3<T4.

The larger the value of the time TZ is, the smaller the value of the exercise menu stage Y is. The smaller the value of the exercise menu stage Y is, the greater the quantity and/or intensity of the exercise menu are. The quantity of the exercise menu and the intensity of the exercise menu are as described above.

Described above is an example in which the lactic acid concentration X,
the exercise menu stage Y, and the exercise menu are associated with each other in the exercise menu database. According to an aspect of the invention it is possible to optionally use a variation XΔ in the lactic acid concentration.

The variation XΔ in the lactic acid concentration is a difference between a lactic acid concentration XT=0 at start of the exercise and the lactic acid concentration XT after start of the exercise. The variation XΔ in the lactic acid concentration may be part of the biological information.

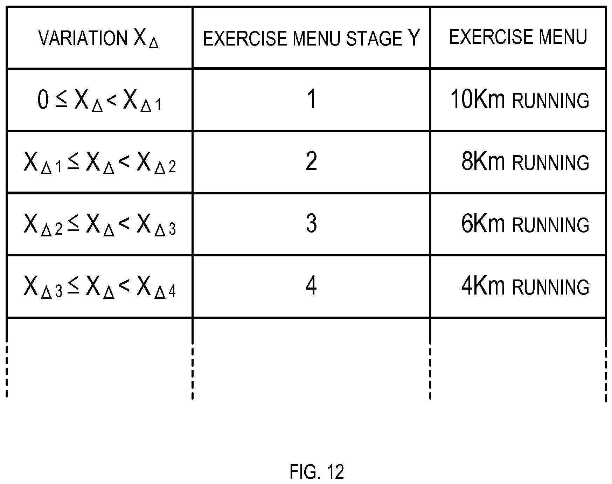

For example, as shown in FIG. 12, when the variation XΔ in the lactic acid concentration is 0 or more and less than XΔ1, the exercise menu stage Y is 1. Further, for example, when the variation XΔ in the lactic acid concentration is XΔ1 or more and less than XΔ2, the exercise menu stage Y is 2. In FIG. 12, a larger/smaller relationship of the numerical values is 0<XΔ1<XΔ2<XΔ3<XΔ4.

The larger the value of the lactic acid concentration XΔ is, the larger the value of the exercise menu stage Y is. The larger the value of the exercise menu stage Y is, the smaller the quantity and/or intensity of the exercise menu are. The quantity of the exercise menu and the intensity of the exercise menu are as described above.

Described above is an example in which the lactic acid concentration X, the exercise menu stage Y, and the exercise menu are associated with each other in the exercise menu database. According to a further aspect of the invention it is possible to optionally use a time TX. The time TX is a period of time from the time point when the exercise is started to a time point when the variation XΔ in the lactic acid concentration reaches a specified threshold. The time TX may be part of the biological information.

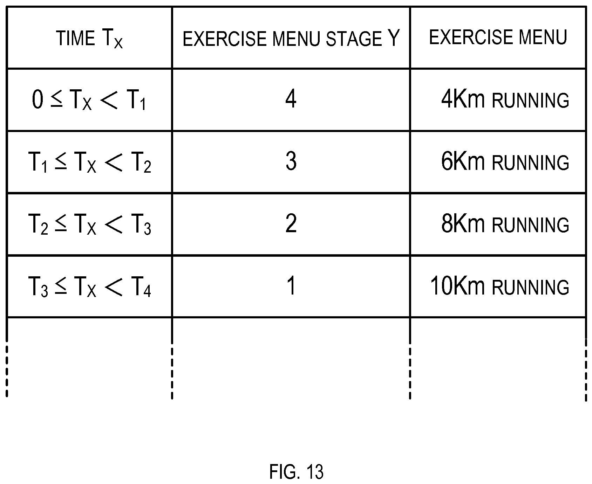

For example, as shown in FIG. 13, when the time TX is 0 or more and less than T1, the exercise menu stage Y is 4. Further, for example, when the time TX is T1 or more and less than T2, the exercise menu stage Y is 3. In FIG. 13, a larger/smaller relationship of the numerical values is 0<T1<T2<T3<T4.

The larger the value of the time Tx is, the smaller the value of the exercise menu stage Y is. The smaller the value of the exercise menu stage Y is, the greater the quantity and/or intensity of the exercise menu are. The quantity of the exercise menu and the intensity of the exercise menu are as described above.

Described above is an example in which the lactic acid concentration X, the exercise menu stage Y, and the exercise menu are associated with each other in the exercise menu database. According to a further aspect of the invention it is possible to optionally use a time TY. The time TY is a period of time from the time point when the exercise is started to a time point when a first-order differential value of the lactic acid concentration with respect to the time first reaches a specified threshold. The time TY may be part of the biological information.

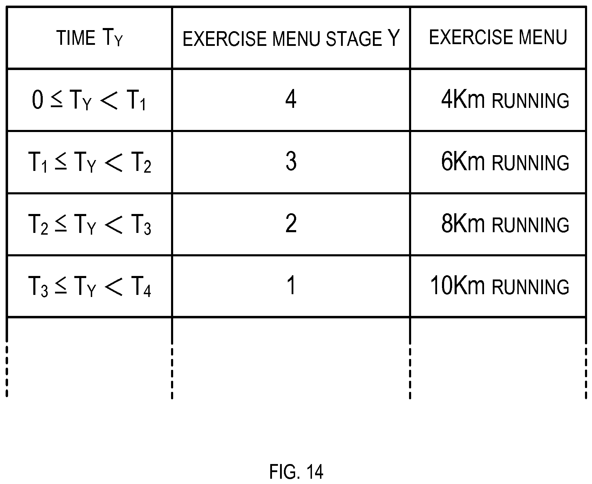

For example, as shown in FIG. 14, when the time TY is 0 or more and less than T1, the exercise menu stage Y is 4. Further, for example, when the time TY is T1 or more and less than T2, the exercise menu stage Y is 3. In FIG. 14, a larger/smaller relationship of the numerical values is 0<T1<T2<T3<T4.

The larger the value of the time TY is, the smaller the value of the exercise menu stage Y is. The smaller the value of the exercise menu stage Y is, the greater the quantity and/or intensity of the exercise menu are. The quantity of the exercise menu and the intensity of the exercise menu are as described above.

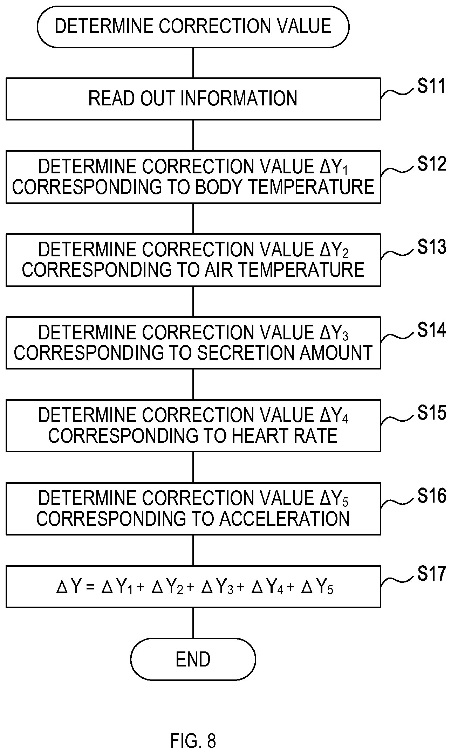

In a step 6, the correction information acquisition unit 39 determines a correction value ΔY. This process will be described with reference to FIG. 8 to FIG. 10.

In a step 11 in FIG. 8, the time information, the body temperature information, the air temperature information, the secretion amount information, the heart rate information, and the acceleration information stored in the memory 31 in the step 2 are read out.

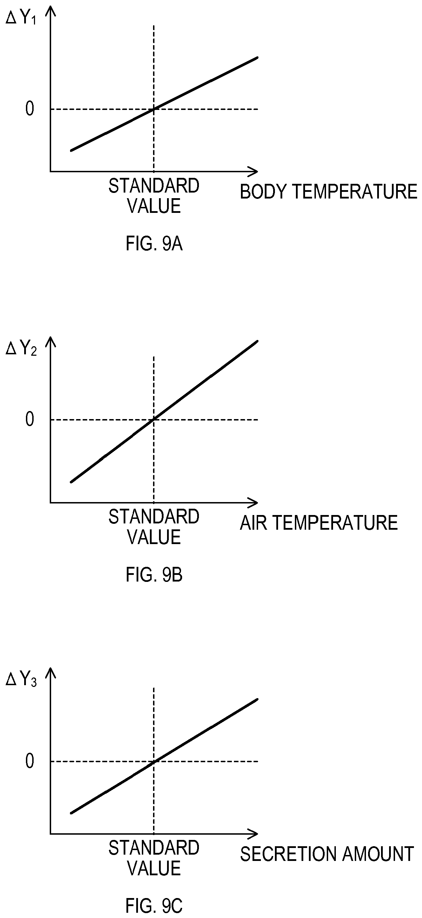

In a step 12, a correction value ΔY1 is determined as below. Stored in advance in the memory 31 is a map specifying a relationship between the body temperature and the correction value ΔY1 as shown in FIG. 9A. In this map, when the body temperature is a standard value, the correction value ΔY1 is 0. When the body temperature is higher than the standard value, the correction value ΔY1 is a positive value, whereas when the body temperature is lower than the standard value, the correction value ΔY1 is a negative value. The greater a difference between the body temperature and the standard value is, the larger the absolute value of the correction value ΔY1 is. The body temperature indicated by the body temperature information read out in the step 11 is applied to this map, to thereby determine the correction value ΔY1.

In a step 13, a correction value ΔY2 is determined as below. Stored in advance in the memory 31 is a map specifying a relationship between the air temperature and the correction value ΔY2 as shown in FIG. 9B. In this map, when the air temperature is a standard value, the correction value ΔY2 is 0. When the air temperature is higher than the standard value, the correction value ΔY2 is a positive value, whereas when the air temperature is lower than the standard value, the correction value ΔY2 is a negative value. The greater a difference between the air temperature and the standard value is, the larger the absolute value of the correction value ΔY2 is. The air temperature indicated by the air temperature information read out in the step 11 is applied to this map, to thereby determine the correction value ΔY2.

In a step 14, a correction value ΔY3 is determined as below. Stored in advance in the memory 31 is a map specifying a relationship between the secretion amount and the correction value ΔY3 as shown in FIG. 9C. In this map, when the secretion amount is a standard value, the correction value ΔY3 is 0. When the secretion amount is larger than the standard value, the correction value ΔY3 is a positive value, whereas when the secretion amount is smaller than the standard value, the correction value ΔY3 is a negative value. The greater a difference between the secretion amount and the standard value is, the larger the absolute value of the correction value ΔY3 is. The secretion amount indicated by the secretion amount information read out in the step 11 is applied to this map, to thereby determine the correction value ΔY3.

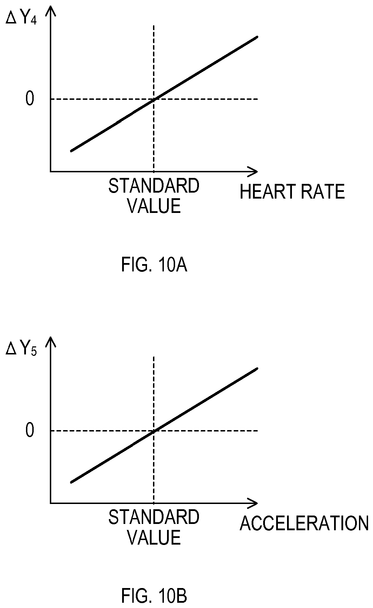

In a step 15, a correction value ΔY4 is determined as below. Stored in advance in the memory 31 is a map specifying a relationship between the heart rate and the correction value ΔY4 as shown in FIG. 10A. In this map, when the heart rate is a standard value, the correction value ΔY4 is 0. When the heart rate is higher than the standard value, the correction value ΔY4 is a positive value, whereas when the heart rate is lower than the standard value, the correction value ΔY4 is a negative value. The greater a difference between the heart rate and the standard value is, the larger the absolute value of the correction value ΔY4 is. The heart rate indicated by the heart rate information read out in the step 11 is applied to this map, to thereby determine the correction value ΔY4.

In a step 16, a correction value ΔY5 is determined as below. Stored in advance in the memory 31 is a map specifying a relationship between the acceleration and the correction value ΔY5 as shown in FIG. 10B. In this map, when the acceleration is a standard value, the correction value ΔY5 is 0. When the acceleration is larger than the standard value, the correction value ΔY5 is a positive value, whereas when the acceleration is smaller than the standard value, the correction value ΔY5 is a negative value. The greater a difference between the acceleration and the standard value is, the larger the absolute value of the correction value ΔY5 is. The acceleration indicated by the acceleration information read out in the step 11 is applied to this map, to thereby determine the correction value ΔY5.

In a step 17, the correction values ΔY1 to ΔY5 determined in the steps 12 to 16 are summed up to determine the correction value ΔY.

The flow returns to FIG. 6, and in a step 7, the exercise menu setting unit 35 corrects the exercise menu by using the correction value ΔY determined in the step 6. Specifically, the following process is performed. First, the correction value ΔY determined in the step 6 is added to the exercise menu stage Y determined in the step 5, to thereby determine a corrected stage Y. When the correction value ΔY is a positive value, the corrected stage Y is larger than the stage Y determined in the step 5. When the correction value ΔY is a negative value, the corrected stage Y is smaller than the stage Y determined in the step 5.

Next, the corrected stage Y is applied to the exercise menu database shown in FIG. 7, to thereby set the exercise menu. This exercise menu is a corrected exercise menu.

In a step 8, the exercise menu presentation unit 37 displays, on the display 27, the exercise menu set in the step 7. "To display on the display 27" corresponds to "to present".

In a step 9, the database creation unit 41 creates a database in which the biological information, the time information, the body temperature information, the air temperature information, the secretion amount information, the heart rate information, the acceleration information, the user's physical information, and the user's lifestyle are associated with one another.

In a step 10, the database creation unit 41 stores, in the memory 31, the database created in the step 9.

### 5. Processes Performed by Sensor Group

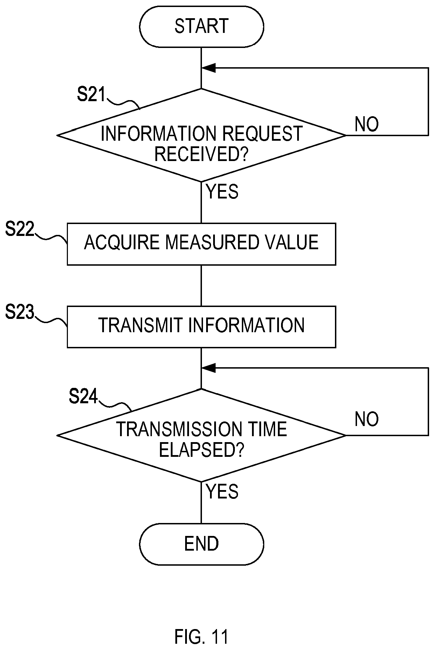

Processes performed by the respective sensors constituting the sensor group will be described with reference to FIG. 11. The processes performed by the respective sensors constituting the sensor group are basically similar to one another. Thus, the process performed by the biosensor 5 will be described here as an example.

In a step 21 in FIG. 11, the controller 45 determines whether the communicator 47 has received the information request. The information request is transmitted by the exercise assistance device 3 in the step 1. If the information request has been received, the process proceeds to a step 22. If the information request has not been received, the process returns prior to the step 21.

In the step 22, the controller 45 acquires a measured value of the lactic acid concentration from the sensor body 43.

In a step 23, the controller 45 transmits information indicating the measured value of the lactic acid concentration acquired in the step 22 (i.e., the biological information) through the communicator 47. The transmitted biological information is received by the exercise assistance device 3.

In a step 24, the controller 45 determines whether a preset transmission time has elapsed from the time point when the information request is received in the step 21. If the transmission time has elapsed, the present process is terminated. If the transmission time has not elapsed yet, the process proceeds to the step 22.

### 6. Effects Achieved by Exercise Assistance Device 3 and Exercise Assistance System 1

(1A) The exercise assistance device 3 can set the exercise menu based on the biological information indicating the lactic acid concentration in the perspiration secreted from the user. This enables appropriate setting of the exercise menu according to the user's condition.

(1B) The exercise assistance device 3 corrects the exercise menu by using the body temperature information, the air temperature information, the secretion amount information, the heart rate information, and the acceleration information. This enables more appropriate setting of the exercise menu according to the user's condition and the surrounding environment.

(1C) The exercise assistance device 3 sets the exercise menu by using the exercise menu database and the biological information. This enables easy setting of the exercise menu.

(1D) The exercise assistance device 3 is a portable information terminal device. This makes it easy to carry around the exercise assistance device 3.

(1E) The exercise assistance device 3 can create the database in which the biological information, the body temperature information, the air temperature information, the secretion amount information, the heart rate information, the acceleration information, the user's physical information, and the user's lifestyle are associated with one another.

### <Other Embodiments>

While an embodiment of the present disclosure has been described so far, the present disclosure is not limited to the above-described embodiment, and can be practiced in variously modified forms.
(1) The information used to correct the stage Y may be a part of the biological information, the body temperature information, the air temperature information, the secretion amount information, the heart rate information, and the acceleration information.
(2) The exercise menu set in the step 5 may be presented in the step 8. That is, the exercise menu set in the step 5 may be used as is without correction.
(3) In the first embodiment, the stage Y is corrected by using the body temperature information, the air temperature information, the secretion amount information, the heart rate information, and the acceleration information. However, the lactic acid concentration X calculated in the step 4 may be corrected by using one or more selected from the body temperature information, the air temperature information, the secretion amount information, the heart rate information, and the acceleration information. Such correction can be made, for example, as below.

Correction is made such that the higher the body temperature is, the higher the lactic acid concentration X is. Correction is made such that the higher the air temperature is, the higher the lactic acid concentration X is. Correction is made such that the larger the secretion amount is, the higher the lactic acid concentration X is. Correction is made such that the higher the heart rate is, the higher the lactic acid concentration X is. Correction is made such that the larger the acceleration is, the higher the lactic acid concentration X is.

Further, it is possible to set the exercise menu by applying the corrected lactic acid concentration X to the exercise menu database shown in FIG. 7 and to present the set exercise menu. Also in this case, the exercise menu can be corrected.
(4) The exercise menu database shown in FIG. 7 may be stored in an element distinct from the exercise assistance system 1. Examples of the element in which the exercise menu database is stored may include a cloud computer. The exercise assistance device 3 can read out the exercise menu database from the cloud computer or the like to use the same.
(5) The exercise assistance device 3 may present the exercise menu in another way. For example, the exercise assistance device 3 may present the exercise menu by voice.
(6) The database created by the exercise assistance device 3 may comprise a part of the time information, the biological information, the body temperature information, the air temperature information, the secretion amount information, the heart rate information, the acceleration information, the user's physical information, and the user's lifestyle.
(7) A function of one element in the above-described embodiments may be shared by multiple elements, and functions of multiple elements may be performed by one element. A part of the elements in the above-described embodiments may be omitted. At least part of the elements in the above-described embodiments may be added to and/or replaced with another element in the above-described embodiments. Any and all modes encompassed by the technical thoughts specified by the language of the claims are embodiments of the present disclosure.
(8) The present disclosure can also be practiced in various forms, other than by the above-described exercise assistance device 3. Examples of such forms are a program to cause a computer to function as the controller 45, a non-transitory tangible storage medium, such as a semiconductor memory, in which this program is recorded, and an exercise assistance method.

## Claims

1. An exercise assistance device (3) comprising:
a biological information acquisition unit (33) configured to acquire biological information from a result of a measurement of lactic acid contained in a secretion from a living body by a biosensor (5);
a storage unit (31) configured to store the biological information;
an exercise menu setting unit (35) configured to set an exercise menu based on the biological information stored in the storage unit; and
an exercise menu presentation unit (37) configured to present the exercise menu set by the exercise menu setting unit,
wherein the biological information is a period of time until a time point when a second-order differential value of the lactic acid concentration with respect to the time first reaches a specified threshold,
wherein the exercise assistance device further comprises
a correction information acquisition unit (39) configured to acquire correction information from a sensor for correction (7, 9, 11, 13, 15) configured to measure one or more selected from a group comprising body temperature of the living body, air temperature, a secretion amount of the secretion, heart rate of the living body, and an acceleration of the living body, the correction information indicating a result of such measurement by the sensor for correction,
wherein the storage unit is configured to store the biological information and the correction information in association with each other, and
the exercise menu setting unit is configured to correct the exercise menu set based on the biological information by using the correction information associated with the biological information.

2. The exercise assistance device according to claim 1,
wherein the correction information comprises the secretion amount of the secretion.

3. The exercise assistance device according to claim 1 or 2,
wherein the secretion is perspiration, and
wherein the biosensor is configured to measure the lactic acid contained in the perspiration, which is the secretion from the living body.

4. The exercise assistance device according to any one of claims 1 to 3,
wherein the biological information further comprises at least one of a variation in lactic acid concentration, a period of time until the variation in the lactic acid concentration reaches a specified threshold, and a period of time until a first-order differential value of the lactic acid concentration with respect to a time first reaches a specified threshold.

5. The exercise assistance device according to any one of claims 1 to 4,
wherein the exercise menu setting unit is configured to set the exercise menu by using an exercise menu database in which the biological information and the exercise menu are associated with each other, and the biological information stored in the storage unit.

6. The exercise assistance device according to any one of claims 1 to 5,
wherein the exercise assistance device is a portable information terminal device.

7. The exercise assistance device according to any one of claims 1 to 6,
wherein the exercise assistance device further comprises a database creation unit (41) configured to create a database comprising the biological information.

8. The exercise assistance device according to claim 7,
wherein the exercise assistance device further comprises an input unit (19) through which input information is inputtable, the input information being one or more selected from a group comprising physical information of the living body, lifestyle information of the living body, and a management ID of the living body, and
wherein the database creation unit is configured to create the database in which the biological information and the input information are associated with each other.

9. An exercise assistance system (1) comprising:
the exercise assistance device according to any one of claims 1 to 8; and
the biosensor.

10. An exercise assistance method comprising:
acquiring biological information from a biosensor (5) that measures lactic acid contained in a secretion from a living body, the biological information indicating a result of such measurement by the biosensor, wherein the biological information is a period of time until a time point when a second-order differential value of the lactic acid concentration with respect to the time first reaches a specified threshold;
acquiring correction information from a sensor for correction (7, 9, 11, 13, 15) that measures one or more selected from a group comprising body temperature of the living body, air temperature, a secretion amount of the secretion, heart rate of the living body, and an acceleration of the living body, the correction information indicating a result of such measurement by the sensor for correction;
storing the biological information and the correction information in association with each other in a storage unit (31);
setting an exercise menu based on the biological information stored in the storage unit;
correcting the exercise menu set based on the biological information by using the correction information associated with the biological information; and
presenting the exercise menu that has been corrected.

11. A non-transitory tangible storage medium in which a program is stored that causes the exercise assistance device of any of claims 1-8 to execute:
a step of acquiring biological information from a biosensor (5) that measures lactic acid contained in a secretion from a living body, the biological information indicating a result of such measurement by the biosensor, wherein the biological information is a period of time until a time point when a second-order differential value of the lactic acid concentration with respect to the time first reaches a specified threshold;
a step of acquiring correction information from a sensor for correction (7, 9, 11, 13, 15) that measures one or more selected from a group comprising body temperature of the living body, air temperature, a secretion amount of the secretion, heart rate of the living body, and an acceleration of the living body, the correction information indicating a result of such measurement by the sensor for correction;
a step of storing the biological information and the correction information in association with each other in a storage unit (31);
a step of setting an exercise menu based on the biological information stored in the storage unit;
a step of correcting the exercise menu set based on the biological information by using the correction information associated with the biological information; and
a step of presenting the exercise menu that has been corrected.

## Patentansprüche

1. Trainingsunterstützungsvorrichtung (3), die Folgendes aufweist:
eine Einheit (33) zur Erfassung biologischer Informationen, die eingerichtet ist, um biologische Informationen aus einem Ergebnis einer Messung von Milchsäure, die in einem Sekret eines lebenden Körpers enthalten ist, durch einen Biosensor (5) zu erfassen;
eine Speichereinheit (31), die zum Speichern der biologischen Informationen eingerichtet ist;
eine Trainingsmenü-Einstelleinheit (35), eingerichtet zum Einstellen eines Trainingsmenüs auf der Grundlage der in der Speichereinheit gespeicherten biologischen Informationen; und
eine Trainingsmenü-Präsentationseinheit (37), die eingerichtet ist, um das durch die Trainingsmenü-Einstelleinheit eingestellte Trainingsmenü zu präsentieren,
wobei die biologischen Informationen eine Zeitspanne bis zu einem Zeitpunkt ist, an dem ein Differentialwert zweiter Ordnung der Milchsäurekonzentration in Bezug auf die Zeit zuerst einen bestimmten Schwellenwert erreicht,
wobei die Trainingsunterstützungsvorrichtung ferner Folgendes aufweist
eine Einheit (39) zur Erfassung von Korrekturinformationen, die eingerichtet ist, um Korrekturinformationen von einem Korrektursensor (7, 9, 11, 13, 15) zu erfassen, der eingerichtet ist, um eines oder mehreres aus einer Gruppe ausgewählt zu messen, die eine Körpertemperatur des lebenden Körpers, eine Lufttemperatur, eine Sekretionsmenge des Sekrets, eine Herzfrequenz des lebenden Körpers und eine Beschleunigung des lebenden Körpers aufweist, wobei die Korrekturinformationen ein Ergebnis einer solchen Messung durch den Korrektursensor anzeigen,
wobei die Speichereinheit eingerichtet ist, um die biologischen Informationen und die Korrekturinformationen in Verbindung miteinander zu speichern, und
die Trainingsmenü-Einstelleinheit eingerichtet ist, um das eingestellte Trainingsmenü auf der Grundlage der biologischen Informationen unter Verwendung der mit den biologischen Informationen verbundenen Korrekturinformationen zu korrigieren.

2. Trainingsunterstützungsvorrichtung nach Anspruch 1,
wobei die Korrekturinformationen die Sekretionsmenge des Sekrets aufweisen.

3. Trainingsunterstützungsvorrichtung nach Anspruch 1 oder 2,
wobei das Sekret Schweiß ist, und
wobei der Biosensor eingerichtet ist, um die im Schweiß enthaltene Milchsäure zu messen, die das Sekret des lebenden Körpers ist.

4. Trainingsunterstützungsvorrichtung nach einem der Ansprüche 1 bis 3,
wobei die biologischen Informationen ferner mindestens eine der folgenden Informationen aufweisen: eine Veränderung der Milchsäurekonzentration, eine Zeitspanne, bis die Veränderung der Milchsäurekonzentration einen bestimmten Schwellenwert erreicht, und eine Zeitspanne, bis ein Differenzialwert erster Ordnung der Milchsäurekonzentration in Bezug auf eine Zeit zuerst einen bestimmten Schwellenwert erreicht.

5. Trainingsunterstützungsvorrichtung nach einem der Ansprüche 1 bis 4,
wobei die Trainingsmenü-Einstelleinheit eingerichtet ist, um das Trainingsmenü unter Verwendung einer Trainingsmenüdatenbank einzustellen, in der die biologischen Informationen und das Trainingsmenü einander zugeordnet sind, und die biologischen Informationen in der Speichereinheit gespeichert sind.

6. Trainingsunterstützungsvorrichtung nach einem der Ansprüche 1 bis 5,
wobei die Trainingsunterstützungsvorrichtung um tragbares Informationsendgerät ist.

7. Trainingsunterstützungsvorrichtung nach einem der Ansprüche 1 bis 6,
wobei die Trainingsunterstützungsvorrichtung ferner eine Einheit (41) zur Erstellung einer Datenbank aufweist, die eingerichtet ist, um eine Datenbank mit den biologischen Informationen zu erstellen.

8. Trainingsunterstützungsvorrichtung nach Anspruch 7,
wobei die Trainingsunterstützungsvorrichtung ferner eine Eingabeeinheit (19) aufweist, über die Eingabeinformationen eingegeben werden können, wobei die Eingabeinformationen eine oder mehrere sind, die aus einer Gruppe ausgewählt sind, die physische Informationen des lebenden Körpers, Lebensstilinformationen des lebenden Körpers und eine Management-ID des lebenden Körpers umfasst, und
wobei die Einheit zur Erstellung der Datenbank eingerichtet ist, um die Datenbank zu erstellen, in der die biologischen Informationen und die Eingabeinformationen einander zugeordnet sind.

9. Trainingsunterstützungssystem (1), das Folgendes aufweist:
die Trainingsunterstützungsvorrichtung nach einem der Ansprüche 1 bis 8; und
den Biosensor.

10. Verfahren zur Trainingsunterstützung, das Folgendes aufweist:
Erfassen biologischer Informationen von einem Biosensor (5), der in einem Sekret aus einem lebenden Körper enthaltene Milchsäure misst, wobei die biologischen Informationen ein Ergebnis einer solchen Messung durch den Biosensor anzeigen, wobei die biologischen Informationen eine Zeitspanne bis zu einem Zeitpunkt sind, an dem ein Differenzialwert zweiter Ordnung der Milchsäurekonzentration in Bezug auf die Zeit zuerst einen bestimmten Schwellenwert erreicht;
Erfassen von Korrekturinformationen von einem Korrektursensor (7, 9, 11, 13, 15), der eines oder mehreres aus einer Gruppe ausgewählt misst, die eine Körpertemperatur des lebenden Körpers, eine Lufttemperatur, eine Sekretmenge des Sekrets, eine Herzfrequenz des lebenden Körpers und eine Beschleunigung des lebenden Körpers aufweist, wobei die Korrekturinformationen ein Ergebnis einer solchen Messung durch den Korrektursensor anzeigen;
Speichern der biologischen Informationen und der Korrekturinformationen in Verbindung miteinander in einer Speichereinheit (31);
Einstellen eines Trainingsmenüs auf der Grundlage der in der Speichereinheit gespeicherten biologischen Informationen;
Korrigieren des Trainingsmenüs, das auf der Grundlage der biologischen Informationen eingestellt ist, unter Verwendung der mit den biologischen Informationen verbundenen Korrekturinformationen; und
Präsentieren des korrigierten Trainingsmenüs.

11. Nicht-transitorisches materielles Speichermedium, in dem ein Programm gespeichert ist, das die Trainingsunterstützungsvorrichtung nach einem der Ansprüche 1-8 dazu bewirkt, folgendes auszuführen:
einen Schritt des Erfassens biologischer Informationen von einem Biosensor (5), der Milchsäure misst, die in einem Sekret eines lebenden Körpers enthalten ist, wobei die biologischen Informationen ein Ergebnis einer solchen Messung durch den Biosensor anzeigen, wobei die biologischen Informationen eine Zeitspanne bis zu einem Zeitpunkt sind, an dem ein Differenzialwert zweiter Ordnung der Milchsäurekonzentration in Bezug auf die Zeit zuerst einen bestimmten Schwellenwert erreicht;
einen Schritt des Erfassens von Korrekturinformationen von einem Korrektursensor (7, 9, 11, 13, 15), der eines oder mehrere aus einer Gruppe ausgewählt misst, die eine Körpertemperatur des lebenden Körpers, eine Lufttemperatur, eine Sekretmenge des Sekrets, eine Herzfrequenz des lebenden Körpers und eine Beschleunigung des lebenden Körpers aufweist, wobei die Korrekturinformationen ein Ergebnis einer solchen Messung durch den Korrektursensor anzeigen;
einen Schritt des Speicherns der biologischen Informationen und der Korrekturinformationen in Verbindung miteinander in einer Speichereinheit (31);
einen Schritt des Einstellens eines Trainingsmenüs auf der Grundlage der in der Speichereinheit gespeicherten biologischen Informationen;
einen Schritt des Korrigierens des Trainingsmenüs, das auf der Grundlage der biologischen Informationen eingestellt ist, unter Verwendung der mit den biologischen Informationen verbundenen Korrekturinformationen; und
einen Schritt der Darstellung des korrigierten Trainingsmenüs.

## Revendications

1. Dispositif d'aide à l'exercice (3) comprenant :
une unité d'acquisition d'informations biologiques (33) configurée pour acquérir des informations biologiques à partir d'un résultat d'une mesure par un biocapteur (5) de l'acide lactique contenu dans une sécrétion d'un corps vivant ;
une unité de stockage (31) configurée pour stocker les informations biologiques ;
une unité de réglage de menu d'exercice (35) configurée pour régler un menu d'exercice en fonction des informations biologiques contenues dans l'unité de stockage ; et
une unité de présentation de menu d'exercice (37) configurée pour présenter le menu d'exercice réglé par l'unité de réglage de menu d'exercice,
les informations biologiques étant une période de temps jusqu'à un instant où une valeur différentielle de second ordre de la concentration d'acide lactique en fonction du temps atteint pour la première fois un seuil spécifié,
le dispositif d'aide à l'exercice comprenant en outre
une unité d'acquisition d'informations de correction (39) configurée pour acquérir des informations de correction auprès d'un capteur de correction (7, 9, 11, 13, 15) configuré pour mesurer un ou plusieurs paramètres sélectionnés parmi un groupe comprenant la température corporelle du corps vivant, la température de l'air, la quantité de sécrétion, le rythme cardiaque du corps vivant et une accélération du corps vivant, les informations de correction indiquant un résultat de ces mesures par le capteur de correction,
l'unité de stockage étant configurée pour stocker les informations biologiques et les informations de correction en association entre elles, et
l'unité de réglage de menu d'exercice étant configurée pour corriger le menu d'exercice réglé en fonction des informations biologiques en utilisant les informations de correction associées aux informations biologiques.

2. Dispositif d'aide à l'exercice selon la revendication 1,
dans lequel les informations de correction comprennent la quantité de sécrétion.

3. Dispositif d'aide à l'exercice selon la revendication 1 ou 2,
dans lequel la sécrétion est la transpiration, et
dans lequel le biocapteur est configuré pour mesurer l'acide lactique contenu dans la transpiration, qui est la sécrétion provenant du corps vivant.

4. Dispositif d'aide à l'exercice selon l'une quelconque des revendications 1 à 3,
dans lequel les informations biologiques comprennent en outre au moins une information parmi une variation de la concentration d'acide lactique, une période de temps jusqu'à ce que la variation de la concentration d'acide lactique atteigne un seuil spécifié, et une période de temps jusqu'à ce qu'une valeur différentielle du premier ordre de la concentration d'acide lactique en fonction du temps atteint pour la première fois un seuil spécifié.

5. Dispositif d'aide à l'exercice selon l'une quelconque des revendications 1 à 4,
dans lequel l'unité de réglage de menu d'exercice est configurée pour régler le menu d'exercice en utilisant une base de données de menu d'exercice dans laquelle les informations biologiques et le menu d'exercice sont associés entre eux, et les informations biologiques stockées dans l'unité de stockage.

6. Dispositif d'aide à l'exercice selon l'une quelconque des revendications 1 à 5,
dans lequel le dispositif d'aide à l'exercice est un dispositif terminal d'information portable.

7. Dispositif d'aide à l'exercice selon l'une quelconque des revendications 1 à 6,
dans lequel le dispositif d'aide à l'exercice comprend en outre une unité de création de base de données (41) configurée pour créer une base de données comprenant les informations biologiques.

8. Dispositif d'aide à l'exercice selon la revendication 7,
dans lequel le dispositif d'aide à l'exercice comprend en outre une unité d'entrée (19) via laquelle des informations peuvent être entrées, les informations d'entrée étant une ou plusieurs informations sélectionnées parmi le groupe comprenant des informations physiques du corps vivant, des informations de style de vie du corps vivant, et un ID de gestion du corps vivant, et dans lequel l'unité de création de base de données est configurée pour créer la base de données dans laquelle les informations biologiques et les informations d'entrées sont associées entre elles.

9. Système d'aide à l'exercice (1) comprenant :
le dispositif d'aide à l'exercice selon l'une quelconque des revendications 1 à 8 ; et
le biocapteur.

10. Procédé d'aide à l'exercice comprenant :
l'acquisition d'informations biologiques auprès d'un biocapteur (5) qui mesure l'acide lactique contenu dans une sécrétion d'un corps vivant, les informations biologiques indiquant un résultat de ces mesures par le biocapteur, les informations biologiques étant une période de temps jusqu'à un instant où une valeur différentielle de second ordre de la concentration d'acide lactique en fonction du temps atteint pour la première fois un seuil spécifié ;
l'acquisition d'informations de correction auprès d'un capteur de correction (7, 9, 11, 13, 15) qui mesure un ou plusieurs paramètres sélectionnés parmi un groupe comprenant la température corporelle du corps vivant, la température de l'air, la quantité de sécrétion, le rythme cardiaque du corps vivant et une accélération du corps vivant, les informations de correction indiquant un résultat de ces mesures par le capteur de correction ;
le stockage des informations biologiques et des informations de correction en association entre elles dans une unité de stockage (31) ;
le réglage d'un menu d'exercice en fonction des informations biologiques stockées dans l'unité de stockage ;
la correction du menu d'exercice réglé en fonction des informations biologiques en utilisant les informations de correction associées aux informations biologiques ; et
la présentation du menu d'exercice qui a été corrigé.

11. Support de stockage tangible non transitoire dans lequel est stocké un programme qui amène le dispositif d'aide à l'exercice selon l'une quelconque des revendications 1 à 8 à exécuter :
une étape d'acquisition d'informations biologiques auprès d'un biocapteur (5) qui mesure l'acide lactique contenu dans une sécrétion d'un corps vivant, les informations biologiques indiquant un résultat de ces mesures par le biocapteur, les informations biologiques étant une période de temps jusqu'à un instant où une valeur différentielle de second ordre de la concentration d'acide lactique en fonction du temps atteint pour la première fois un seuil spécifié ;
une étape d'acquisition d'informations de correction auprès d'un capteur de correction (7, 9, 11, 13, 15) qui mesure un ou plusieurs paramètres sélectionnés parmi un groupe comprenant la température corporelle du corps vivant, la température de l'air, la quantité de sécrétion, le rythme cardiaque du corps vivant et une accélération du corps vivant, les informations de correction indiquant un résultat de ces mesures par le capteur de correction ;
une étape de stockage des informations biologiques et des informations de correction en association entre elles dans une unité de stockage (31) ;
une étape de réglage d'un menu d'exercice en fonction des informations biologiques stockées dans l'unité de stockage ;
une étape de correction du menu d'exercice réglé en fonction des informations biologiques en utilisant les informations de correction associées aux informations biologiques ; et
une étape de présentation du menu d'exercice qui a été corrigé.
